## Europäisches Patentamt

## European Patent Office

## Office européen des brevets

(19)

(11) Numéro de publication: **0 099 783**
**B1**

# FASCICULE DE BREVET EUROPÉEN

(12)

(45) Date de publication du fascicule du brevet:
24.09.86

(51) Int. Cl.⁴: **A 61 F 5/02**

(21) Numéro de dépôt: **83401318.7**

(22) Date de dépôt: **24.06.83**

(54) Lombostat à assemblage d'éléments semi-rigides avec une pelote gonflable.

(30) Priorité: **01.07.82 FR 8211555**

(43) Date de publication de la demande:
**01.02.84 Bulletin 84/5**

(45) Mention de la délivrance du brevet:
**24.09.86 Bulletin 86/39**

(84) Etats contractants désignés:
**AT BE CH DE GB IT LI LU NL SE**

(56) Documents cité:
**FR-A-2 355 494**
**GB-A-2 086 712**
**US-A-3 871 367**
**US-A-4 202 327**

(73) Titulaire: **S.A. ARLUX, Le Croisy, F-44700 Orvault (FR)**

(72) Inventeur: **Freland, Jean- Claude, Les Hauts de Basse- Goulaine, F-44120 Basse- Goulaine (FR)**
Inventeur: **Desffains, Jean- Paul, 5, rue des Glénans, F-44700 Orvault (FR)**
Inventeur: **Valle, Roger, 1, rue des Irlandais, F-44800 Saint Herblain (FR)**
Inventeur: **Wallez, Bernard, 155 Route des Vallées, F-44120 Basse- Goulaine (FR)**
Inventeur: **Batard, Denis, 25 Avenue Parc de Procé, F-44100 Nantes (FR)**

(74) Mandataire: **Lemonnier, André, 4 Boulevard Saint- Denis, F-75010 Paris (FR)**

## Description

La présente invention concerne les corsets orthopédiques dits lombostats utilisés pour le traitement préventif et correctif des affections lombaires orthopédiques et rhumatologiques.

On a déjà proposé dans le FR-A-2.290.884 correspondant au US-A-3.871.367 de préfabriquer les corsets orthopédiques de ce type en réalisant un certain nombre de moules symétriques de différentes tailles constitués par une couche externe en une matière plastique dure à peu près rigide et une couche interne en une matière plastique compressible, la coquille étant fendue selon sa ligns axiale arrière avec des moyens de fixation liberables fixes sur les portions postérieures adjacentes.

Ce type de lombostat nécessite un nombre de tailles de moules important en raison de la faible capacité d'adaptation à la corpulence de l'utilisateur, il est difficile à mettre en place et à serrer par l'utilisateur lui-même en raison de la position dorsale des moyens de fixation libérables, et enfin il est peu efficace pour assurer une auto-élongation du rachis. En outre ce lombostat présente des pelotes lombaires de volumes fixes.

On a également proposé dans le FR-A-2.355-494 de réaliser ces corsets par un gilet en tissu inextensible qui enveloppe le tronc avec des moyens de réglage pour l'ajuster au corps, ce gilet comportant des structures gonflables de raidissement fixées l'une sur le dos, l'autre sur le devant dans l'axe du gilet avec une poche abdominale gonflable placée contre la cavité abdominale.

Ce type de corset présente une rigidité insuffisante et n'assure pas un appui sous-costal nettement marqué utile pour l'autoélongation du rachis.

La présente invention qui concerne un lombostat du type décrit dans US-A-4.202.327 qui est réalisé par un assemblage d'éléments semi-rigides constitué par deux demi-coquilles latérales, c'est-à-dire s'adaptant aux côtés du torse humain et réalisées en matière plastique semi-rigide, a pour but de fournir un lombostat de ce type qui est adaptable à une gamme élargie de corpulences, qui bénéficie de la rigidité inhérente à un tel type de coquille, dont l'ajustement et le réglage du serrage sur le patient sont réglables facilement par celui-ci même, dont la forme assure une augmentation de la pression intraabdominale et une élongation du rachis et dont le volume des pelotes lombaires est réglable.

La présente invention a également pour but de fournir un lombostat qui soit solide, esthétique, facile d'emploi et d'entretien et léger.

Ces buts sont atteints, conformément à l'invention, par le fait que les deux demi-coquilles sont réunies le long des bords dorsaux adjacents par une bande mince semi-rigide formant pont, avec des moyens de solidarisation et de serrage entre les bords avant adjacents des demi- coquilles et qu'une pelote lombaire gonflable formant deux pelotes symétriques par rapport à l'axe longitudinal du lombostat est maintenue en position par une lame rigide qui est fixée dans la bande mince semi-rigide formant pont.

Selon une autre caractéristique de l'invention, la bande flexible mince formant pont est appliquée sur la surface extérieure arrière des deux demi-coquilles dont les bords présentent un certain écartement de manière à ménager une gouttière selon l'axe des apophyses épineuses. La bande flexible est solidarisée avec les demi-coquilles par rivetage, collage ou tout autre moyen de fixation assurant une résistance suffisante au cisaillement. L'utilisation d'une solidarisation amovible, par exemple par bandes du tissu à bouclettes et crochets vendu sous la marque commerciale déposée "VETCRO", permet un réglage ultérieur et éventuellement une réutilisation.

Selon une autre caractéristique la pelote lombaire gonflable a une forme en violon avec un lobe inférieur plus important. Selon un mode de réalisation préférentiel la pelote lombaire est subdivisée en cavités ou canaux de direction principale verticale dont la section va en décroissant de la ligne axiale vers les zones périphériques. De préférence la pelote lombaire est associée avec une lame rigide, notamment métallique, disposée sur sa face tournée vers l'intérieur et elle est solidarisée avec la bande mince semi-rigide formant pont au droit de la gouttière des apophyses épineuses. Cette solidarisation peut se faire par rivetage sur la bande flexible des extrémités de la lame rigide.

La valve de gonflage peut être disposée en un point quelconque par exemple à la partie inférieure de la pelote gonflable.

Selon une autre caractéristique les demi-coquilles comportent des ouvertures latérales de forme elliptique en bas et en avant au niveau des crêtes iliaques. Ces ouvertures facilitent l'adaptation des demi-coquilles et le réglage du pince taille. au niveau trochantérien.

La forme d'ensemble des demi-coquilles est caractérisée par une partie de ceinture concave destinée à s'engager entre les crêtes iliaques et la partie sous-costale latérale, tout en respectant la zone épigastrique et par une surface antérieure abdominale concave.

Selon un mode de réalisation préférentiel, les moyens de solidarisation et de serrage entre les bords avant adjacents des deux demi-coquilles sont constitués par des sangles fixées au voisinage du bord antérieur de l'une des demi-coquilles, par des anneaux ou organes de renvoi analogues des sangles fixés au voisinage du bord antérieur de l'autre demi-coquille et par des moyens de fixation des extrémités des sangles disposés au voisinage du bord antérieur de la première demicoquille. Les moyens de fixation sont de préférence du type permettant un serrage réglable et, par exemple, du type "VELCRO" ou du type boucle à pincement. Les moyens de renvoi pour les sangles peuvent être constitués par des boucles rectangulaires montées à rotation autour de leur point de fixation pour admettre une petite différence de niveau entre les deux demi-coquilles mais il est également possible, pour réduire les saillies sur la surface antérieure, de les réaliser par des lumières à bord renforcé réalisées dans la surface de la demi-coquille.

On décrira ci-après un mode de réalisation du lombostat conforme à l'invention avec référence au dessin ci-annexé dans lequel:

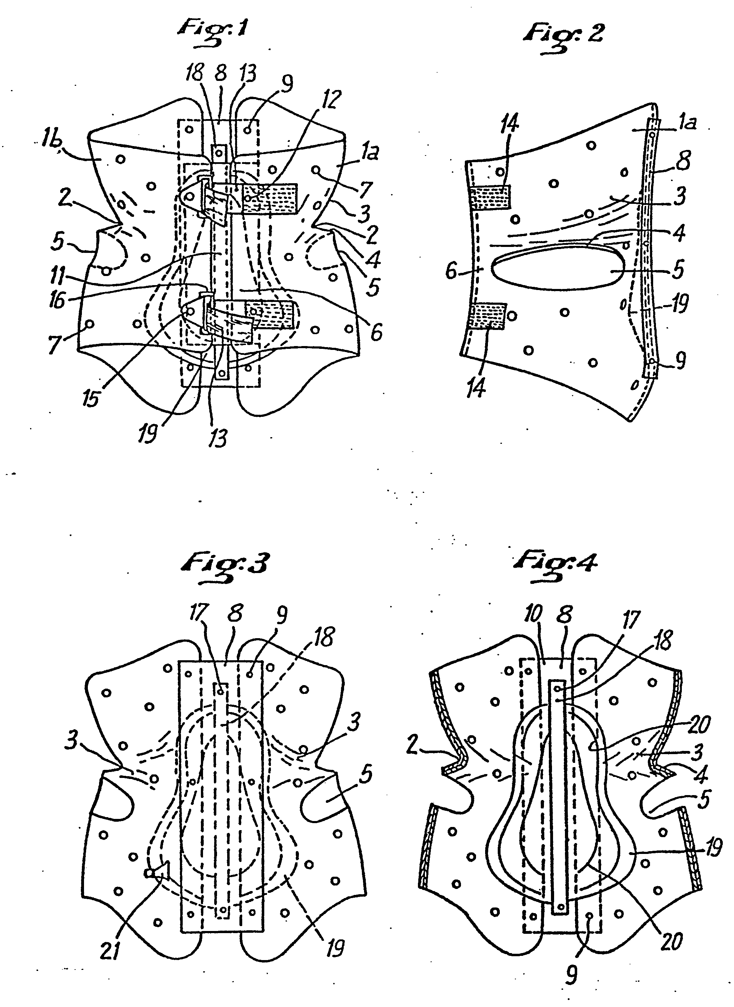

La figure 1 est une vue en élévation par l'avant du lombostat; la figure 2 en est une vue en élévation latérale; la figure 3 en est une vue par l'arrière et la figure 4 une vue en coupe par un plan frontal vertical.

Le lombostat comporte deux demi-coquilles 1a et 1b avec une zone concave formant pince taille 2 qui sépare un soutien sous-costal 3 d'une zone 4 d'appui sur la crête iliaque. En dessous de la zone 4 est réalisée une large ouverture 5 de forme sensiblement elliptique pour dégager la crête iliaque. La partie avant 6 est concave pour assurer une pression intra-abdominale. Comme illustré à la figure 4 ces demi-coquilles sont de préférence réalisées en deux couches, une couche externe en thermo-plastique rigide et une couche interne en mousse, en tissu ou autre. Des perforations 7 qui peuvent être remplacées par des ajours de section plus importante assurent une aération.

Ces demi-coquilles sont réalisées en un nombre réduit de tailles qui répondent à toutes les corpulences. Le réglage par l'appareilleur est possible du fait que les deux demi-coquilles 1a et 1b sont solidarisés par une bande en manière plastique mince semi-rigide 8 qui est solidarisée avec les deux demi-coquilles, par exemple par des rivets 9 ou par collage, les deux demi-coquilles 1 laissant entre leurs bords arrière une gouttière 10 ayant une largeur d'au moins deux centimètres pour éviter l'appui direct sur les apophyses epineuses.

A l'avant une lame en matière plastique mince et flexible 11 est fixée sur la face interne voisine du bord de l'une des demi-coquilles 1a, par exemple par des rivets 12, et lorsque le lombostat est mis en place elle est engagée derrière la demi-coquille 1b. Sur la face frontale de la demi-coquille 1a sont fixées deux sangles 13 dont la surface comporte un élément 14 en le produit vendu sous la marque commerciale déposée "VELCHO". Sur l'autre demi-coquille 1b sont fixées, par des rivets 15, des boucles rectangulaires 16 dans lesquelles peuvent être engagées les sangles 13 qui après serrage sont fixées par application de la surface en le produit vendu sous la marque commerciale déposée "VELCHO" prévue sur leur face interne sur la surface 14. Ce mode de réalisation des moyens de fixation permet un réglage extrèmement précis du serrage, à la différence des boucles à ardillon, et le rivet de fixation 15 permet une rotation de la boucle 16 lorsque les deux demicoquilles sont légèrement décalées.

Dans la gouttière 10 est d'autre part fixée, par exemple par des rivets 17, une barre rigide 18 de préférence en métal qui maintient la rigidité des différents éléments et assure le maintien en position de la pelote lombaire gonflable 19. Comme illustré à la figure 4 cette pelote gonflable à une forme en violon. Elle est réalisée en pellicule thermoplastique ou autre et elle est subdivisée par des soudures 20 en des canaux de direction générale verticale avec des sections decroissant du centre vers la périphérie. Une valve 21 permet de gonfler la pelote et de régler son volume en fonction des variations de poids du patient et de l'évolution du traitement tout en assurant une compression lombaire.

Le lombostat ainsi réalisé permet d'assurer une élongation vraie du rachis grâce aux trois points d'élongation formés par la compression abdominale en 6, l'appui sous-costal en 3 et la compression lombaire assurée par la pelote gonflable 19.

## Revendications

1. Lombostat à assemblage d'éléments semi-rigides, constitué par deux demi-coquilles latérales (1a, 1b), c'est-à-dire s'adaptant aux côtés du torse humain et réalisées en matière plastique semi-rigide, caractérisé en ce que les deux demi-coquilles (1a, 1b) sont réunies le long des bords dorsaux adjacents par une bande mince semi-rigide (8) formant pont, avec des moyens de solidarisation et de serrage (13) entre les bords avant adjacents des deux demi-coquilles et en ce qu'une pelote lombaire gonflable (19) formant deux pelotes symétriques par rapport à l'axe longitudinal du lombostat, est maintenue en position par une lame rigide (18), qui est fixée dans la bande (8).

2. Lombostat selon la revendication 1, caractérisé en ce que la bande mince semi-rigide formant pont (8) est appliquée sur la surface extérieure arrière des deux demi-coquilles (1a, 1b) dont les bords présentent un certain écartement de manière à ménager une gouttière (10) selon l'axe des apophyses épineuses.

3. Lombostat selon l'une quelconque des revendications 1 et 2, caractérisé en ce que la pelote lombaire gonflable (19) a une forme en violon avec un lobe inférieur plus important.

4. Lombostat selon la revendication 3, caractérisé en ce que la pelote lombaire (19) est subdivisée en cavités ou canaux de direction principale verticale dont la section va en décroissant de la ligne axiale vers les zones périphériques.

5. Lombostat selon l'une quelconque des revendications 1 à 4, caractérisé en ce que la pelote lombaire (19) est associée avec la lame rigide (18), notamment métallique, disposée sur sa face tournée vers l'intérieur, ladite pelote étant solidarisée avec la bande mince semi-rigide (8) formant pont au droit de la gouttière des apophyses épineuses.

6. Lombostat selon l'une quelconque des revendications 1 à 5,

caractérisé en ce que chaque demi-coquille (1a-1b) comporte une ouverture latérale (5) de forme elliptique en bas et en avant au niveau des crêtes iliaques.

7. Lombostat selon l'une quelconque des revendications 1 à 6,

caractérisé en ce que chaque demi-coquille (1a-1b) comporte une partie de ceinture concave (2-3-4) destinée à s'engager entre les crêtes iliaques et la partie sous-costale latérale, tout en respectant la zone épigastrique et une surface antérieure abdominale (6) concave.

8. Lombostat selon l'une quelconque des revendications 1 à 7,

caractérisé en ce que les moyens de solidarisation et de serrage entre les bords avant adjacents des deux demi-coquilles sont constitués par des sangles (13) fixées au voisinage du bord antérieur de l'une des demi-coquilles, par des anneaux (16) ou organes de renvoi des sangles analogues fixés au voisinage du bord antérieur de l'autre demi-coquille et par des moyens de fixation (14) des extrémités des sangles disposés au voisinage du bord antérieur de la première demi-coquille.


**Patentansprüche**

1. Aus halbfesten Teilen zusammensetzbares Hüftkorsett, das aus zwei seitlichen Halbschalen (1a, 1b) gebildet ist, d.h. daß sie sich an die Seiten des menschlichen Rumpfes anpassen und daß sie aus halbsteifem Kunststoffmaterial gebildet sind,

dadurch gekennzeichnet, daß die zwei Halbschalen (1a, 1b) entlang der aneinanderliegenden Rückenkanten durch einen dünnen halbsteifen Streifen (8) verbunden sind, der als Brücke dient, mit Mitteln für die Verbindung und den Verschluß (13) zwischen den vorderen aneinander liegenden Kanten der zwei Halbschalen und dadurch, daß ein aufblasbares Kreuzkissen (19), das zwei in Bezug auf die Längsachse des Hüftkorsetts symmetrische Kissen bildet, in Position durch ein steifes Blatt (18) gehalten ist, das am Streifen (8) fixiert ist.

2. Hüftkorsett nach Anspruch 1, dadurch gekennzeichnet, daß der dünne halbsteife Streifen (8), der als Brücke dient, an der hinteren Außenfläche der zwei Halbschalen (1a, 1b) angebracht ist, deren Kanten einen bestimmten Abstand in der Art bilden, um eine Rinne (10) gemäß der Achse der Dornfortsätze auszusparen.

3. Hüftkorsett nach einem der Ansprüche 1 und 2, dadurch gekennzeichnet, daß das aufblasbare Kreuzkissen (19) die Form einer Geige aufweist, mit einem unteren, breiteren Lappen.

4. Hüftkorsett nach Anspruch 3, dadurch gekennzeichnet, daß das Kreuzkissen (19) durch Aushöhlungen oder Kanäle von hauptsächlich vertikaler Ausrichtung unterteilt ist, deren Querschnitt von der axialen Linie zu den peripheren Zonen abnehmend verläuft.

5. Hüftkorsett nach einem der Ansprüche 1 bis 4, dadurch gekennzeichnet, daß das Kreuzkissen (19) mit dem steifen, insbesondere metallischem Blatt (18) verbunden ist, wobei es an seiner nach innen gerichteten Fläche angeordnet ist, wobei besagtes Kissen mit dem dünnen, halbsteifen Streifen (8) verbunden ist, der als Brücke zufolge der Rinne für die Dornfortsätze dient.

6. Hüftkorsett nach einem der Ansprüche 1 bis 5, dadurch gekennzeichnet, daß jede Halbschale (1a, 1b) eine seitliche Öffnung (5) von elliptischer Form am Rücken und vorne am Niveau der Hüftknochen aufweist.

7. Hüftkorsett nach einem der Ansprüche 1 bis 6, dadurch gekennzeichnet, daß jede Halbschale (1a, 1b) eine konkave Gürtelpartie (2, 3, 4), die bestimmt ist, um sich zwischen dem Hüftknochen und der seitlichen Partie unterhalb der Rippen anzulegen, insbesondere zur Schonung der Magengrube und eine vordere bauchseitige, konkave Fläche (6) aufweist.

8. Hüftkorsett nach einem der Ansprüche 1 bis 7, dadurch gekennzeichnet, daß die Mittel für die Verbindung und den Verschluß zwischen den beiden vorderen aneinanderliegenden Kanten der zwei Halbschalen durch Gurtbänder (13), die in der Nähe der vorderen Kante der einen der Halbschalen angebracht sind, durch Ringe (16) oder Rückhaltelemente für die Gurtbänder, die analog in der Nähe der vorderen Kante der anderen Halbschale fixiert sind, und durch Mittel für die Fixierung (14) der Enden der Gurtbänder, die in der Nähe der vorderen Kante der ersten Halbschale angeordnet sind, gebildet sind.


**Claims**

1. An orthopaedic corset made of the assembly of semi-rigid elements, formed of two side half-shells (1a, 1b), that is which adapt themselves to the sides of the torso of a human body and made of a semi-rigid plastic material,

wherein the two half-shells (1a, 1b) are connected along the adjacent dorsal edges by a semi-rigid thin band (8) forming a bridge, with attachment and tightening means (13) between the adjacent front edges of the two half-shells and an inflatable lumbar pad (19) forming two symmetrical pads relative to the corset longitudinal axis, is maintained in position by a rigid bar (18), which is fixed in the band (8).

2. An orthopaedic corset according to claim 1, wherein the semi-rigid thin band forming a bridge (8) is applied

on the rear outer surface of the two half-shells (1a, 1b), the edges of which are spread apart so as to provide a gutter (10) along the spinous processes.

3. An orthopaedic corset according to any one of claims 1 and 2, wherein the inflatable lumbar pad (19) has the shape of a violoin, with an enlarged inferior lobe.

4. An orthopaedic corset according to claim 3, wherein the lumbar pad (19) is subdivided into cavities or channels in a general vertical direction, the section of which is decreasing from the axial line to the peripheral zones.

5. An orthopaedic corset according to any one of claims 1 to 4, wherein the lumbar pad (19) is associated with the rigid bar (18), notably metallic, disposed on its face turned inwardly, said pad being rigidly connected to the semi-rigid thin band (8) forming a bridge opposite the gutter of the spinous processes.

6. An orthopaedic corset according to any one of claims 1 to 5, wherein each half-shell (1a, 1b) is formed of a side opening (5) of elliptical shape at the bottom and in the front, at the level of the iliac crests.

7. An orthopaedic corset according to any one of claims 1 to 6, wherein each half-shell (1a, 1b) comprises a concave waist portion (2-3-4) to be engaged between the iliac crests and the side sub-costal region, while respecting the epigastric zone and a concave abdominal anterior surface (6).

8. An orthopaedic corset according to any one of claims 1 to 7, wherein the attachment and tightening means between the adjacent front edges of the two half-shells are made of straps (13) fixed in the vicinity of the anterior edge of one of the half-shells, by rings (16) or similar return members of the straps fixed in the vicinity of the anterior edge of the other half-shell and by fixation means (14) of the ends of the straps disposed in the vicinity of the anterior edge of the first half-shell.

Fig. 1

Fig. 2

Fig. 3

Fig. 4